Europäisches Patentamt

European Patent Office

Office européen des brevets

(11) Publication number: **0 303 756**
**A2**

## (12) EUROPEAN PATENT APPLICATION

(21) Application number: **88101434.4**

(22) Date of filing: **02.02.88**

(51) Int. Cl.⁴: **A61M 25/00**

(30) Priority: **17.08.87 US 85551**

(43) Date of publication of application:
**22.02.89 Bulletin 89/08**

(84) Designated Contracting States:
**AT BE CH DE ES FR GB IT LI NL SE**

(71) Applicant: **B. Braun Melsungen AG**
**Carl-Braun Strasse**
**D-3508 Melsungen(DE)**

(72) Inventor: **Griffin, Joseph C.**
**221 Almira Avenue**
**Atco New Jersey 08004(US)**
Inventor: **Skaggs, James L.**
**98 Burnt House Road**
**Indian Mills New Jersey 08088(US)**

(74) Representative: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Thermodilution and pressure transducer balloon catheter.**

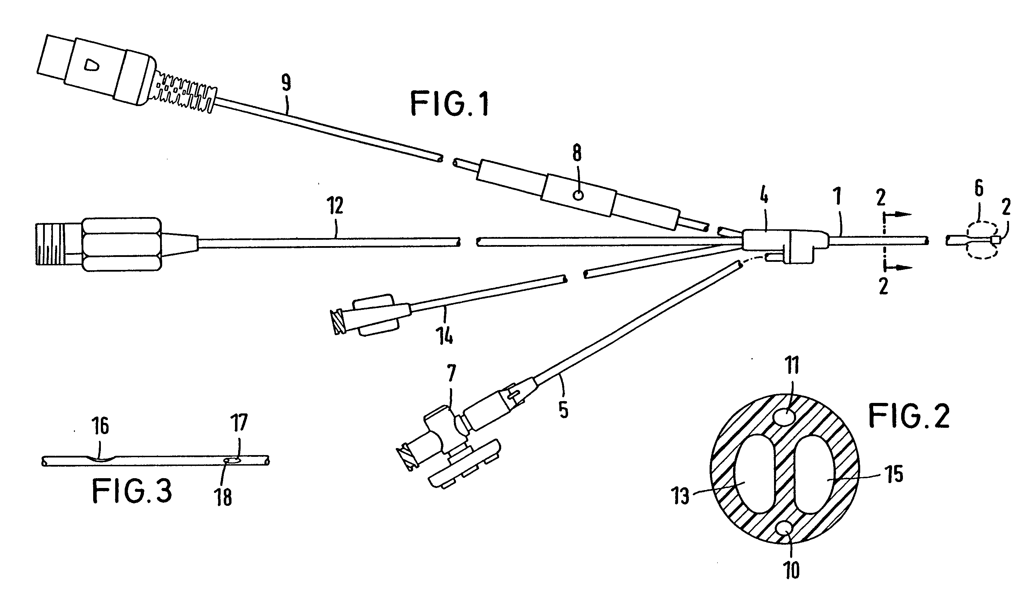

(57) The catheter (1) of the present invention employs a forward facing pressure sensor (2) mounted at the distal tip of a balloon catheter. The pressure sensor (2), so oriented as shown in FIG. 1 avoids both the hydraulic coupling systems previously used and the described problems attendant their use, as well as the inaccuracies which can be encountered with the use of laterally facing strain gauge pressure sensors. The pressure readings obtained demonstrates no overshooting or mechanical resonance found in conventional catheters with connecting tubing for hydraulic coupling to an external dome transducer.

FIG. 1

FIG. 2

## THERMODILUTION AND PRESSURE TRANSDUCER BALLOON CATHETER

### Background of the Invention

#### Field of the Invention

The present invention pertains to balloon catheters which are useful in performing pulmonary artery catheterization and more particularly to multi-lumen balloon catheters which are capable of measuring intravascular and intracardiac pressure precisely as well as being capable of being used for thermodilution studies. .

#### Description of the Prior Art

Typically, balloon catheters such as the Swan-Ganz thermodilution catheter have been used to obtain certain important cardiac diagnostic information. A single catheter insertion could be used for measuring pulmonary artery and pulmonary capillary wedge pressures, right atrial pressure as well as sampling from either the right atrium or pulmonary artery, as well as injection of a cold solution and detection of temperature change for a determination of cardiac output. In one example of such a catheter which has been widely used, a quadruple lumen seven french catheter body with a balloon at the distal end was structured with a large lumen terminating at the tip of the catheter. This lumen was employed for obtaining pulmonary artery pressure or pulmonary capillary wedge pressure using a fluid coupling. Another lumen terminated in an injection orifice approximately 30 cm proximal to the distal tip of the catheter which provided for injection of cold solution into the right atrium or superior vena cava when the catheter tip was positioned in the pulmonary artery. Central venous pressure could also be measured through this lumen as an alternative to injecting a cold solution. Another lumen was provided for gaseous balloon inflation and the last lumen was provided to carry the electrical leads to the thermistor temperature detector which was exposed at the surface of the catheter about 3.5 cm proximal to the tip.

The direct method of measuring blood pressure provided by the Swan-Ganz catheter employed a hydraulic coupling system which exhibited several disadvantages in use. The principal disadvantage has been termed overshoot since the fluid coupling tended to produce a lag in the measurement which could mask or obscure valuable information and, in some situations, could produce a pressure reading that gave a false indication of a functional condition.

Likewise, movement of the catheter would produce variations in readings from mechanical resonance that could be misinterpreted. Attempts to overcome these problems have been attempted. For example, catheter tip pressure transducers, such as disclosed in U.S. Patent No. 4,274,423 have employed rectangular cantilever beam type pressure sensitive diaphragms and strain gauges which were sufficiently miniaturized to measure vessel blood pressure radially of the catheter tip. Such devices have utility but are, however, subject to distortions in the pressure readings due to the resiliency of the blood vessel walls adjacent the transducer location, and their design limits their use to catheters without a balloon.

Other devices, such as shown in U.S. Patent No. 3,550,583 represented attempts to overcome the drawbacks of the previous design for measurement of blood pressure without the use of external hydraulic connectors. The described transducer assembly had limited utility as it was incorporated into an injection needle shape which was not adapted for use in a balloon catheter structure.

It is therefore an object of the present invention to provide a balloon catheter with a axially disposed pressure transducer which in use can provide accurate, nearly instantaneous readings of capillary pressure or pulmonary artery occluded pressure during pulmonary artery catheterization and can provide accurate pressure measurements anywhere in the right side of the heart that can be reached with the catheter.

### Summary of the Invention

The balloon catheter of the present invention is provided with a pressure transducer tip assembly which, in combination with thermodilution study capability provides the objects and advantages of the present invention. A pressure sensor mounted on the distal tip operates on the strain gauge principal. This miniature transducer is designed to function in the vascular system on the tip of a balloon catheter thereby eliminating the hydraulic coupling system necessary with most pressure measuring balloon catheters. The catheter of the present invention will operate in conjunction with other functions of a balloon catheter such as thermodilution studies, blood sampling, or drug infusion. The pressure transducer is preferably a silicon strain gauge that is characterized by its property of changing electrical resistance as a function of pressure. The strain gauge itself is contained within a stainless steel housing and covered with a blood compatible silicone rubber diaphragm. It is contemplated, however, that any axially disposed transducer, properly sized, can be employed. In the preferred embodiment small copper wires connected to the sensor strain gauge pass through a lumen within the catheter body and are capable of transmitting a signal from the strain gauge to the proximal end of the catheter. An interfacing cable designed to match the transducer to a preselected conventional pressure monitoring equipment, provide for pressure measurement, and recording.

### Brief Description of the Drawings

Fig. 1 is a side elevation view of one embodiment of the balloon catheter of the present invention.

Fig. 2 is a cross-sectional view of the balloon catheter of Fig. 1 taken at the lines and arrows 2-2 of Fig. 1.

Fig. 3 is a broken section of the distal portion of the catheter of Fig. 1 showing that catheter with thermodilution capability.

Fig. 4 is a broken partially sectioned view of the balloon tip of the catheter shown in Fig. 1 before the transducer is fully in place.

Fig. 5 is a partly broken view of the balloon tip of the catheter shown in Fig. 1 with the transducer in place.

Fig. 6 is a partially sectioned view of the balloon tip shown in Fig. 5 in an inflated or extended position.

Fig. 7 is a broken view of another embodiment of the catheter of the present invention.

Fig. 8 is a cross-sectional view of a five lumen embodiment of the present invention.

Fig. 9 is a cross-sectional view of a two lumen embodiment of the present invention.

Fig. 10 is a cross-sectional view of a three lumen embodiment of the present invention.

### Detailed Description of the Invention

The present invention avoids the drawbacks previously described which were characteristic of the prior catheters employing both direct and indirect measurement techniques. The catheter of the present invention employs a forward facing pressure sensor mounted at the distal tip of a balloon catheter. The pressure sensor, so oriented as shown in the drawings, avoids both the hydraulic coupling systems previously used and the described problems attendant their use, as well as the inaccuracies which can be encountered with the use of laterally facing strain gauge pressure sensors. The pressure readings obtained with the device of the present invention demonstrates no overshooting or mechanical resonance found in conventional catheters with connecting tubing for hydraulic coupling to an external dome transducer.

Further, the placement or location of the balloon with respect to the transducer at the tip, whose face is substantially perpendicular to the longitudinal axis of the catheter, enables the user greater ease of insertion and manipulation. This contributes to more accurate placement and therefore more precise measurements of direct impact pressure in the wedge position as well as correct direct blood pressure measurements during placement, regardless of the patient's position. Likewise, no further zeroing after the initial calibration before insertion is required.

In its preferred embodiment, the catheter of the present invention employs a transducer which comprises a silicon strain gauge housed within an axially oriented stainless steel cylindrical housing containing a forward facing blood-compatible silicone rubber diaphragm or a similar compliant material. The transducer is connected to copper wires sized to pass through one lumen of the catheter. The strain gauge employed exhibits changes in electrical resistance as a function of pressure. The sensed resistance changes are transformed by conventional equipment into calibrated pressure readings on appropriate equipment.

Fig. 1 shows a partial plan view of one embodiment of the balloon catheter of the present invention. The catheter body 1 is generally in excess of about 100 cm in length and is extruded from materials which are blood compatible. In addition, the catheter is marked with distance stripes around the catheter at 10 cm interval. These stripes are preferably coded as to number and thickness to represent distance from the distal end of the catheter to enable a visual indication of the depth of the catheter at the insertion site. The transducer assembly 2, described in more detail hereinafter, is disposed perpendicular to the longitudinal axis of the catheter so as to face longitudinally of a vessel instead of laterally. The multiple lumen catheter body terminates at its proximal end with a manifold 4 into which tubes or wires are inserted to communicate with the interior lumens of the catheter body 1 and is provided internally with sufficient hollow space or spaces (not shown) so that appropriate external connections can be provided to each individual lumen of the tubes and the lumens of the catheter body 1.

Referring to Fig. 2, a typical four lumen catheter cross-section is shown. In this embodiment the lumen 10 is provided. When properly connected to a gas supply tube 5, the lumen 10 is thereby provided with means for introducing a source of gas, preferably $CO_2$, to the balloon for inflation, when desired. The supply tube 5 is fitted with a valve means 7 which includes a removable fitting or fixture for closing the balloon 6 from the source of gas (not shown) while in an inflated condition, removing the supply tube 5 from the source of gas, if desired, and controllably releasing the gas pressure inside of the balloon 6 to deflate the balloon when desired.

The lumen 11 in Fig. 2 is provided for accommodating conducting electrical leads and thermistor for temperature sensing, as will be more fully described hereinafter. Electrical connector and lead assembly 12 is provided to communicate with lumen 11.

Another connector such as a LuerLock™ type of connector and proximal tube assembly 14 is provided for fluid tight communication with proximal lumen 15 (Fig. 2), whose thermodilution function will be more fully described hereinafter.

Transducer lead and connector assembly 9 is provided for electrical connection with transducer 2 through lumen 13, Fig. 2.

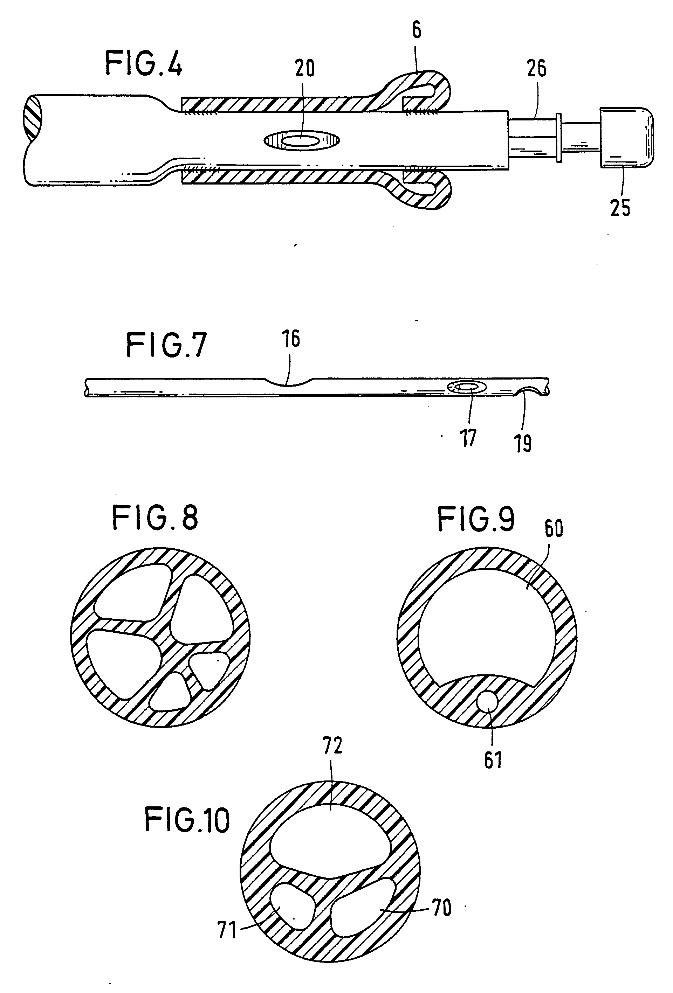

Referring to Figs. 8, 9 and 10, alternative cross-sectional embodiments of the catheter of the present invention are shown. In Fig. 9, only two lumens are provided. Lumen 60 is provided for accommodating the electrical connections as a transducer assembly 2, and lumen 61 is provided for communicating with a source of gas under pressure for inflating and deflating the balloon 6 as previously described for a four lumen pressure transducer catheter.

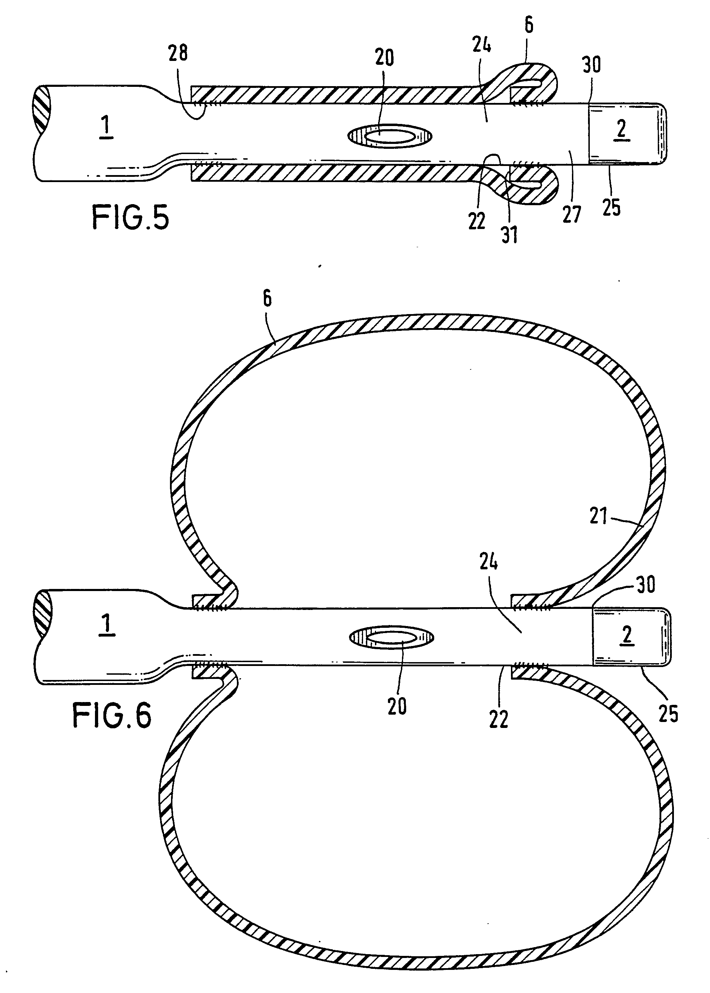

In Fig. 10 only three lumens are provided. Lumen 70 is provide for accommodating the electrical connections as a transducer assembly 2, and lumen 71 is provided for communicating with a source of gas under pressure for inflating and deflating the balloon 6 as previously described for a four lumen pressure transducer catheter. Lumen 72 can be used with appropriate connecting assemblies for blood sampling, drug infusion, or the like.

In Fig. 8 a typical cross-section of a five lumen catheter is shown. Beside accommodating all of the functions of a four lumen catheter an additional lumen can be used with appropriate connecting assemblies for blood sampling, drug infusion, or the like.

Referring to Figs. 4 and 5, the exit opening 20 from lumen 10 is shown exhausting into the space defined by balloon 6. Referring to Fig. 6 the balloon 6 is shown in its approximate fully inflated position which is radially outward of the catheter body 1 and provides an annulus 21 around the housing of transducer 2.

In the inflated condition shown in Fig. 6, the balloon and transducer 2, when positioned in the appropriate locations in a vessel or artery, are uniquely suited for impact and wedge pressure measurements which are obtained in pulmonary artery catheterization for their diagnostic value in evaluating heart function and damage.

The extension 26 from transducer 2 (Fig. 4) is provided so as to be adhesively received in lumen 13 after the distal tip of catheter body 1 has been necked down as shown in Figs. 4, 5, and 6. The forward or distal end 31 of the balloon sleeve is received onto the catheter tip and then curled back onto itself so that it can be adhesively adhered to the outside of the catheter 1 through what would normally be the outside of the balloon sleeve. The outside of the balloon sleeve is adhesively attached to the necked down portion of the catheter body and then rolled proximally for subsequent adhesive attachment to the outside proximal portion of the necked down distal tip at 28. The balloon 6 is sized to be large enough to cover the gas lumen opening 20 in a manner which will enable inflation of the balloon in the manner show in Fig. 6. This assembly provides for a balloon configura tion, when inflated, which symmetrically aligns the transducer 2 within the artery or vessel for more accurate measurement of blood pressure.

The transducer 2 shown in Figs. 4, 5, and 6 is comprised of a disc which is received inside of the transducer head or body 25 and held in position by flange and diaphragm inside the transducer head (not shown). The diaphragm is made of a flexible complaint material such as silicone elastomer or similar material which transmits the force of the fluid (blood) from the face of disc onto a strain gauge element which is preferably a silicon piezoelectric element which when distorted or deflected changes in electrical resistance. This change in resistance is typically measured by a conventional wheatstone bridge circuit and related directly to blood pressure which is displayed in any convenient manner during use. Electrical leads are provided to connect the strain gauge through the lumen 13, and the transducer lead assembly and connector 9 to the appropriate instrumentation (not shown) for display of the pressure sensed.

Transducer vent 8 is provided to vent the lumen to the atmosphere. This allows for equalization of pressure on both sides of the silicon strain gauge to reduce the effect of barometric pressure on the measurement of resistance. Zero base-line will not then shift with changes in barometric pressure.

Referring now to Fig. 3 and Fig. 1 of the preferred embodiments of the present invention is provided with the capacity for making thermodilution measurements. Thermodilution is an application of the calorimetric principle that, in a mixture of fluids at different temperatures, the heat lost by one fluid equals the heat gained by the other. For each fluid, the mathematical product of the temperature change, specific heat and mass is equal.

A recognized method for the study of blood circulation involves producing a temperature change in the blood at one point in the blood flow and measuring the temperature change at a second downstream point. Assuming that the measurement of the temperature change occurs at a point downstream of the heat source and that the blood's heat content is uniform, the measured change will reflect the amount of blood passing through the blood vessel.

In thermodilution studies heat is ether removed from or added to the blood stream. One technique involves the injection of a cooler saline solution into the blood. In use, a known amount of a cold solution at a known temperature is injected into the right atrium or superior vena cava as through a hole 16 in the catheter 1 body which communicates with a source of fluid at a known temperature and the resultant temperature when mixed with the blood is detected by the thermistor 17 (Fig. 3) while the catheter is placed so that the thermistor 17 is in the pulmonary artery, cardiac output is inversely proportional to the integral of the observed temperature change. The accuracy of this method is dependent upon the accuracy of the measurement of the temperature of the injectate and the accuracy of the measured temperature of the resultant blood-injectate mixture. Assuming that the blood's heat content is uniform, the measured change in temperature provides a means of calculating the mass of the blood moving in a specific period of time and therefore the amount of blood flowing through the vessel which is a measure of the cardiac output of a particular patient.

Likewise, when properly placed and the balloon deflated, pulmonary artery pressure can be measured by the forward facing strain gauge without the variations that attend the use of fluid coupled pressure measuring devices or side facing strain gauges. The latter experience problems due to the transducer strain gauge diaphragm coming into direct contact with the vessel wall and the variable resilience of the vessel walls which can expand and contract. When the balloon is inflated pulmonary wedge pressure can be recorded and interpreted.

This particular configuration in addition insures the proper orientation of the transducer diaphragm facing axially of the artery or vein when the balloon is inflated. The inflated balloon is symmetrical and the transducer will be more or less equidistant from the vessel walls.

The connector and tube assembly 14 is provided to join in fluid tight relationship with proximal injection lumen 15, which is provided with a proximal opening 16. The connector and tube assembly 14 is provided with a source of fluid at a known temperature which is injected into the catheter 1 at a known rate and exhausted through proximal opening 16 into the vessel into which the catheter is placed when in use. The lumen 15 is preferably blocked distally of the proximal opening 16. The thermistor opening 17 which communicates with the electrical connector and tube assembly 12 is provided with a plug or seal into which is received a thermistor bead 18 which is connected electrically by wires through the bead lumen 11 to the thermistor bead. The structure is connected to appropriate temperature display means (not shown) by means of electrical connections with the electrical connector and tube assembly 12, to thereby enable a user to calculate the volume of blood flowing by measuring the difference in temperature between the volume of fluid introduced and the temperature of the mixed fluid and blood at the locus of the thermistor bead 18 at the distal locations.

An additional lumen, such as shown in Fig. 8, can be provided to enable infusion of other fluids during the catheterization procedures. Fig. 7 shows the approximate preferred location of such an additional infusion port 19, which is preferably about 2 cm proximal from the distal tip. The thermistor bead 18 is located approximately 3.5 cm proximally from the distal tip.

It will be appreciated that variations in structure can be employed with the catheter combination described which will be within the scope of this invention which is to be limited only to the appended claims as limited by pertinent prior art.

## Claims

1. A multiple lumen balloon catheter for pulmonary artery catheterization procedures comprising:
a multiple lumen catheter body having a distal end portion and a proximal portion;
inflatable balloon means surrounding said distal portion and including hollow elastic cylindrical sleeve means having inside and outside surfaces, said sleeve means adhesively affixed proximal to said distal end to said catheter body by the inside surface of said sleeve means and adhesively affixed adjacent to the distal end of said catheter body by the outside surface of said sleeve means which has been turned inwardly of the sleeve so as to be inside at least a portion of the inside surface of said sleeve means, and

transducer means received in the distal end portion of said catheter body and adhesively affixed thereto, said transducer means having a deformable distal face generally perpendicular to the longitudinal axis of said catheter body, said face being capable of being deflected in response to fluid pressure, and further including strain gauge means communicating with said deformable distal face in a manner so as to be capable of exhibiting changes in electrical resistance in response to movements of said deformable distal face, said transducer means being located axially of said balloon means when said balloon means is inflated.

2. The multiple lumen catheter of claim 1 wherein said multiple lumen catheter further contains infusion means, said infusion means including another of said lumens, said lumen of said infusion means communicating with the outside of said catheter body at a first locus proximal to said distal tip and said lumen further provided to communicate with a source of infusion fluid at a second location proximal to said first locus.

3. The multiple lumen catheter of claim 1 wherein said multiple lumen catheter body further contains at least one additional lumen and temperature sensing means said temperature sensing means including thermistor means located in said lumen and electrical measuring means, said electrical measuring means including connecting means connected to said thermistor means and to means for measuring and displaying temperatures sensed by said thermistor means.

4. The multiple lumen catheter of claim 2 wherein said thermistor means is located adjacent to the outer periphery of said catheter body at a position distal to said locus of communication of said infusion means lumen with the outside of said catheter body.

5. The multiple lumen catheter of claim 2 wherein said catheter comprises at least four lumens.

6. The multiple lumen catheter of claims 3 wherein said catheter comprises at least five lumens.

7. A multiple lumen balloon catheter for pulmonary artery catheterization procedures comprising:

a multiple lumen catheter body having a distal end portion and a proximal portion;

inflatable balloon means surrounding said distal portion and including hollow elastic cylindrical sleeve means having inside and outside surfaces, said sleeve means adhesively affixed proximal to said distal end to said catheter body by the inside surface of said sleeve means and adhesively affixed adjacent to the distal end of said catheter body by the outside surface of said sleeve means which has been turned inwardly of the sleeve so as to be inside at least a portion of the inside surface of said sleeve means, and

transducer means received in the distal end portion of said catheter body and adhesively affixed thereto, said transducer means having a deformable distal face generally perpendicular to the longitudinal axis of said catheter body, said face being capable of being deflected in response to fluid pressure, and further including strain gauge means communicating with said deformable distal face in a manner so as to be capable of exhibiting changes in electrical resistance in response to movements of said deformable distal face, said transducer means being located axially of said balloon means when said balloon means is inflated, and

infusion means including at least another of said lumens said infusion means being exposed to the outside of said catheter body proximal to said distal tip for introducing or receiving fluid therethrough, and

temperature sensing means in at least one lumen exposed to the outside of said catheter body between said exposed infusion lumen and said distal tip for measuring the temperature of the fluid external to said catheter body at the location of said temperature sensing means.

FIG.1

FIG.2

FIG.3

9

8

12

4 1 2

6 2

2

14

7

5

11

13 15

10

16 17

18

EP 0 303 756 A2

FIG.4

FIG.7

FIG.8

FIG.9

FIG.10

FIG.5

FIG.6